# EUROPÄISCHE PATENTSCHRIFT

(11) **EP 1 119 634 B1**
(45) Veröffentlichungstag und Bekanntmachung des Hinweises auf die Patenterteilung: **26.05.2004**
(21) Anmeldenummer: 99941616.7
(22) Anmeldetag: 13.08.1999
(51) Int. Cl.: C12P 41/00, C12P 13/02

(54) **ENZYMKATALYSIERTE RACEMATSPALTUNG VON PRIMÄREN AMINEN**
ENZYME-CATALYZED RACEMIC CLEAVAGE OF PRIMARY AMINES
DISSOCIATION DES RACEMATES D'AMIDES PRIMAIRES CATALYSEE PAR ENZYME

(30) Priorität: 20.08.1998 DE 19837745
(43) Veröffentlichungstag der Anmeldung: 01.08.2001
(73) Patentinhaber: BASF AKTIENGESELLSCHAFT, 67056 Ludwigshafen (DE)
(72) Erfinder: NÜBLING, Christoph, D-67454 Ha loch (DE); DITRICH, Klaus, D-67161 Gönnheim (DE); DULLY, Christian, D-67069 Ludwigshafen (DE)
(86) Internationale Anmeldenummer: PCT/EP1999/005958
(87) Internationale Veröffentlichungsnummer: WO 2000/011203

(56) Entgegenhaltungen:
- EP-A- 0 890 649
- WO-A-97/28271
- US-A- 5 728 876
- B. ORSAT ET AL.: "Homocarbonates a Substrates for the Enantioselective Enzymatic Protection of Amines." J. AM. CHEM. SOC., Bd. 118, 1996, Seiten 712-713, XP002079158
- V. M. SANCHEZ ET AL.: "Candida antarctica lipase catalyzed resolution of ethyl (+/-) -3-aminobutyrate." TETRAHEDRON: ASYMMETRY, Bd. 8, Nr. 1, 1997, Seiten 37-40, XP004015173

## Beschreibung

Die vorliegende Erfindung betrifft ein Verfahren zur Racematspaltung von alkoxysubstituierten primären Aminen durch Umsetzung mit einem Ester in Gegenwart einer Lipase, anschließender Trennung des gebildeten optisch aktiven Amids vom optisch aktiven, nicht umgesetzten Amin. Gegebenenfalls schließt sich die Hydrolyse des optisch aktiven Amids, Trennung des dabei entstehenden optisch aktiven Amins von der dem Ester zugrunde liegenden Säure, Racemisierung und Rückführung des unerwünschten Enantiomers des Amins sowie Veresterung und Rückführung der Säure an.

In WO 95/08636 wird ein Verfahren zur Racematspaltung primärer und sekundärer Amine durch Umsetzung mit einem Ester in Gegenwart von Hydrolasen, insbesondere Lipasen, beschrieben. Bevorzugte Amine sind primäre Arylalkylamine. In WO 96/23894 wird ein Verfahren zur Racematspaltung primärer und sekundärer heteroatomsubstituierter Amine durch Umsetzung mit einem Ester in Gegenwart von Hydrolasen, insbesondere Lipasen, beschrieben. Bevorzugte Amine sind O-geschützte Aminoalkohole. In beiden Anmeldungen werden als bevorzugte Ester die C₁₋₄-Alkylester der C₁₋₄-Alkoxyessigsäuren genannt.

In DE 196 03 575 bzw. DE 196 37 336 wird ein Verfahren zur Herstellung von optisch aktiven Aminen durch Umsetzung der entsprechenden Racemate mit einem Ester in Gegenwart von Lipase aus Candida antarctica beschrieben. Bevorzugte Amine sind alkoxysubstituierte Alkylamine, insbesondere 2-Amino-1-methoxypropan, bzw. substituierte Phenylethylamine, insbesondere 4-Chlor-phenylethylamin. Bevorzugte Ester sind C₁₋₆-Alkansäureester bzw. C₁₋₈-Alkoxyalkansäureester, insbesondere Methoxyessigsäuremethylester. Schließlich wird in DE 196 21 686 ein Verfahren zur Herstellung von optisch aktiven Aminen durch Umsetzung der entsprechenden Racemate mit einem Ester in Gegenwart von Hydrolasen beschrieben, in dem substituierte Phenylethylamine, insbesondere 4-Chlor-phenylethylamin, und C₁₋₄-Halogenalkansäureester, insbesondere Chloressigsäureethylester, bevorzugt sind.

Überraschenderweise wurde nun gefunden, daß das eingangs beschriebene Verfahren besonders vorteilhaft durchgeführt werden kann, wenn Ester mit langkettigen Alkoholresten verwendet werden.

Das Verfahren zur Racematspaltung von Aminen läuft besonders vorteilhaft, wenn ein Ester der allgemeinen Formel 1, 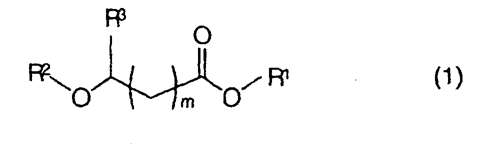 in der
- m =: 0 oder 1,
- R¹ =: verzweigtes oder unverzweigtes C₆-C₂₀-Alkyl oder Heteroalkyl mit 6 bis 20 Gerüstatomen,
- R² =: C₁-C₈-Alkyl oder Phenyl,
- R³ =: H oder C₁-C₄-Alkyl bedeuten,
mit einem alkoxy oder benzyloxy substituierten primären Amin der allgemeinen Formel 2, 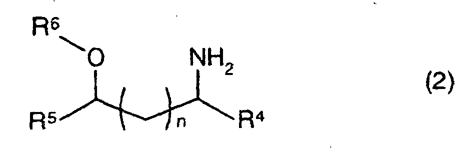 in der
- n =: 0 oder 1,
- R⁴, R⁵: unabhängig voneinander H, C₂-C₈-Alkyl oder Phenyl,
- R⁶ =: C₁-C₆-Alkyl oder Benzyl bedeuten,
in Gegenwart einer Lipase umgesetzt wird,
wobei Amide der allgemeinen Formel 3 entstehen, 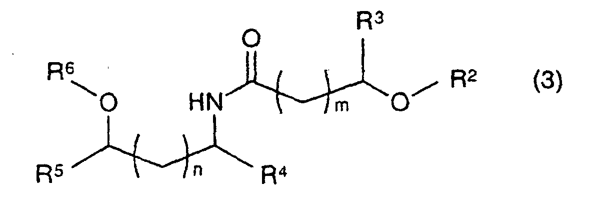 in der
- n, m, R², R³, R⁴, R⁵ und R⁶: die oben angegebenen Bedeutungen haben,
die einen Überschuß an einem optischen Isomeren des Amids aufweisen.

Bevorzugt wird ein (R)-Amid gebildet, besonders bevorzugt wird enantioselektiv acyliert mit hohem Enatiomerenüberschuß von mehr als 50 % ee, besonders mehr als 80 % ee, insbesondere mehr als 90 % ee. Das nicht umgesetzte Amin weist einen Überschuß an (S)-Amin auf, bevorzugt mehr als 50 % ee, besonders von mehr als 90 % ee, insbesondere von mehr als 99 % ee. Unter (R)-Amid oder (R)-Amin werden diejenigen optisch aktiven Amide oder Amine verstanden, die am Kohlenstoff der Aminogruppe eine (R)-Konfiguration aufweisen. Analoges gilt für (S)-Amid oder (S)-Amin.

Weiterhin wurde gefunden, daß es vorteilhaft ist, die Einsatzstoffe zu trocknen. Dies kann prinzipiell in jeder dem Fachmann bekannten Weise erfolgen, z.B. durch azeotrope Trocknung oder durch Trockenmittel wie Natriumsulfat, Magnesiumsulfat, KOH, Phosphorpentoxid, Molekularsieb, Kieselgel oder Aluminiumoxid.

Darüber hinaus wurde gefunden, daß es vorteilhaft ist, säurefreie Einsatzstoffe zu verwenden. Säuren können prinzipiell in jeder dem Fachmann bekannten Weise entfernt werden, z.B. durch Extraktion oder Destillation, gegebenenfalls nach vorheriger Neutralisation mit Alkali- oder Erdalkalihydroxiden wie z.B. Natrium-, Kalium- oder Calciumhydroxid, mit Aminen wie z.B. Triethylamin, Tributylamin, Triethanolamin, Pyridin oder N,N-Dimethylanilin, mit Carbonaten wie z.B. Natrium-, Kaliumoder Calciumcarbonat oder mit Ionenaustauschern.

Es können eine Vielzahl von Lipasen in dem erfindungsgemäßen Verfahren eingesetzt werden. Bevorzugt sind mikrobielle Lipasen aus Bakterien, wie Lipasen aus den Gattungen Bacillus oder Pseudomonas, wie z.B. Amano P oder die Lipase aus Pseudomonas spec. DSM 8246, oder aus Pilzen wie Aspergillus oder Hefen, wie Candida. Weitere bevorzugte Lipasen sind z.B. die von Novo Nordisk kommerziell erhältlichen Lipasen SP 523, SP 524, SP 525, SP 526 und Novozym® 435, die aus Pilzen wie Humicola, Mucor oder Candida antartica gewonnen werden. Außerdem können die von Boehringer Mannheim kommerziell erhältlichen Lipasen Chirazyme L1, L2, L3, L4, L5, L6, L7 und L8 eingesetzt werden. Die Lipasen können in nativer oder immobilisierter Form eingesetzt werden. Die immobilisierte Lipasen können mikroverkapselt sein, mit Präpolymerisaten emulgiert und polymerisiert sein, mit bifunktionellen Substanzen (Oligomeren, Aldehyden etc.) vernetzt sein oder an anorganische oder organische Trägermaterialien, wie z.B. Celite, Lewatit, Zeolithe, Polysaccharide, Polyamide oder Polystyrolharze, gebunden vorliegen. Besonders bevorzugt sind die Lipasen Novozym® 435 und Chirazyme L2.

Die enzymkatalysierte Racematspaltung kann sowohl in protischen oder aprotischen Lösungsmitteln als auch lösungsmittelfrei durchgeführt werden. Geeignete Lösungsmittel sind z.B. Kohlenwasserstoffe wie Hexan, Cyclohexan oder Toluol, Ether wie z.B. Diethylether, Dioxan, Methyl-tert.-butylether, tert.-Amyl-methylether oder THF, Nitrile, wie Acetonitril, Butyronitril, Alkohole, wie tert.-Butanol, 3-Methyl-3-pentanol, und halogenierte Kohlenwasserstoffe wie z.B. Methylenchlorid.

Die Reaktion mit Lipase findet im Allgemeinen bei Atmosphärendruck statt, gegebenenfalls unter Inertgas, wie Stickstoff oder Argon. Sie kann aber auch unter erhöhtem Druck ausgeführt werden.

Die Temperatur für die Umsetzung des Esters mit dem racemischen alkoxysubstituierten Amin liegt üblicherweise bei 0 bis 90°C, bevorzugt bei 10 bis 60°C, besonders bevorzugt bei 20 bis 50°C.

Pro Mol racemischem Amin werden 0,5 bis 2,0 Mol, bevorzugt 0,5 bis 1 Mol Ester eingesetzt. Die erforderliche Enzymmenge hängt von der Aktivität der Enzympräparation und der Reaktivität des Amins ab und kann leicht durch Vorversuche ermittelt werden. In der Regel werden 0,1 bis 10 Gew.-%, bevorzugt 1 bis 5 Gew.-% der immobilisierte Enzympräparation (bezogen auf das racemische Amin) eingesetzt. Novozym® hat eine Aktivität von etwa 7000 U/g bei der Veresterung von Laurinsäure mit 1-Propanol.

Der Reaktionsverlauf läßt sich leicht mit üblichen Methoden wie GC oder HPLC verfolgen. Wenn der gewünschte Umsatz erreicht ist, wird die Reaktion vorzugsweise durch Entfernen des Katalysators, beispielsweise durch Abfiltrieren des (geträgerten) Enzyms, beendet. Die Reaktion kann beispielsweise auch durch Zugabe von Enzym zerstörenden Substanzen wie Säuren oder Laugen oder durch Erhitzen gestoppt werden. Bei kontinuierlicher Fahrweise kann der Umsatz über die Belastung des Enzyms, d.h. der Menge Amin, die pro Zeiteinheit durch den Enzymreaktor gepumpt wird, geregelt werden. Das Verfahren kann vorzugsweise kontinuierlich geführt werden, aber auch batch-weise oder semi-kontinuierlich durchgeführt werden.

Am Ende der enzymkatalysierten Racematspaltung liegt ein Gemisch aus dem acylierten Amin-Enantiomer, dem nicht umgesetztem Amin-Enantiomer, dem bei der Acylierung aus dem Ester freigesetzten Alkohol und evtl. im Überschuß eingesetztem Ester vor. Zur Trennung dieses Gemischs eignen sich insbesondere destillative und extraktive Verfahren. So können niedrig siedende Amine direkt aus dem Reaktionsgemisch abdestilliert werden. Das Amid läßt sich anschließend destillativ oder extraktiv vom Alkohol und gegebenenfalls Ester abtrennen und kann dann in üblicher Weise sowohl sauer als auch basisch, beispielsweise durch Kochen mit Schwefelsäure bzw. Natron- oder Kalilauge, unter Racemisierung oder auch ohne Racemisierung hydrolysiert werden. Die Hydrolyse kann bei Normaldruck, zur Beschleunigung der Reaktion gegebenenfalls auch bei höherer Temperatur unter erhöhtem Druck durchgeführt werden. Das bei der Hydrolyse gebildete zweite Amin-Enantiomer kann, gegebenenfalls nach Freisetzung aus dem Ammoniumsalz, destillativ oder extraktiv isoliert werden. Die bei der Hydrolyse gebildete Säure kann, gegebenenfalls nach Ansäuern der Hydrolyselösung und vorzugsweise extraktiv zurückgewonnen werden. Die Säure kann nach üblichen Verfahren, z.B. azeotrop oder extraktiv, verestert und in das Racematspaltungsverfahren zurückgeführt werden.

Sollte nur ein Enantiomer des Amins benötigt werden, so kann das andere racemisiert und das Racemat in das Verfahren zurückgeführt werden. Auf diese Weise kann theoretisch das gesamte Racemat in das gewünschte Enantiomer überführt werden. Solche Racemisierungen können z.B. unter denselben Bedingungen, unter denen Amine aus Alkoholen oder Ketonen hergestellt werden ("reduktive Aminierung"), durchgeführt werden.

Für das erfindungsgemäße Verfahren eignen sich Ester der Formel 1, 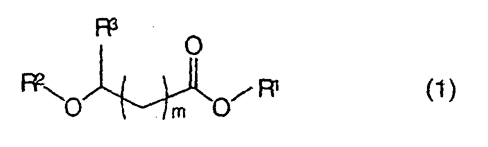 in der
- m =: 0 oder 1, bevorzugt 0
- R¹ =: verzweigtes oder unverzweigtes C₆-C₂₀-Alkyl oder Heteroalkyl mit 6 bis 20 Gerüstatomen, wobei der Alkyl oder Heteroalkylrest unabhängig voneinander durch 1 bis 5 Halogenatome, bevorzugt F oder Cl und/oder eine Oxogruppe substituiert sein kann. Unter Heteroalkyl wird verstanden, daß 1, 2 oder 3 nicht benachbarte -CH₂-Gruppen durch -O-, -S-, -NH-, oder 1 oder 2 nicht benachbarte CH-Gruppe durch N ersetzt sind. Bevorzugt ist der Ersatz von 1 oder 2 CH₂-Gruppen. Das bevorzugte Heteroatom ist O. Beispiele für R¹ sind 1-Hexyl, 2-Hexyl, 3-Hexyl, 2-Methyl-1-pentyl, 3-Methyl-1-pentyl, 4-Methyl-1-pentyl, 2-Methyl-3-pentyl, 2-Ethyl-1-butyl, 1-Heptyl, 2-Heptyl-, 3-Heptyl-, 2-Methyl-1-hexyl, 3-Methyl-1-hexyl, 2-Ethyl-1-hexyl, 3-Ethyl-1-hexyl, 2-Methyl-1-heptyl, 3-Methyl-1-heptyl, 1-Octyl, 2-Octyl-, 3-Octyl-, 1-Nonyl, 2-Nonyl-, 3-Nonyl, 1-Decyl, 2-Decyl, 3,7-Dimethyl -1-octyl, 1-Undecyl, 1-Dodecyl, 1-Tridecyl, 1-Tetradecyl, 1-Pentadecyl, 1-Hexadecyl, 1-Heptadecyl und 1-Octadecyl, 2-Methoxyacetoxyethyl, 2-Methoxyacetoxybutyl, 2-Methoxyacetoxyhexyl, 2-Methoxyacetoxydecyl, Chloroacetoxybutyl, Chloroacetoxyhexyl, Chloroacetoxydecyl, Trichloroacetoxybutyl, Trichloroacetoxyhexyl, Trichloroacetoxydecyl, bevorzugt sind verzweigtes oder unverzweigtes C₆-C₁₈-Alkyl oder Heteroalkyl mit 6 bis 18 Gerüstatomen, wie z.B. 1-Hexyl, 1-Heptyl, 2-Ethyl-1-hexyl, 1-Octyl, 1-Decyl, 1-Dodecyl, 1-Tetradecyl, 1-Hexadecyl und 1-Octadecyl, 2-Methoxyacetoxyethyl, 2-Methoxyacetoxybutyl, 2-Methoxyacetoxyhexyl, 2-Methoxyacetoxydecyl, Chloroacetoxybutyl, Chloroacetoxyhexyl, Chloroacetoxydecyl, besonders bevorzugt sind verzweigtes oder unverzweigtes C₆-C₁₄-Alkyl oder Heteroalkyl mit 6 - 14 Gerüstatomen, z.B.: 1-Hexyl, 2-Ethyl-1-hexyl, 1-Octyl, 1-Decyl und 1-Tetradecyl, 2-Methoxyacetoxyethyl, 2-Methoxyacetoxybutyl, 2-Methoxyacetoxyhexyl, 2-Methoxyacetoxydecyl.
- R² =: C₁-C₈-Alkyl wie z.B. Methyl, Ethyl, 1-Propyl, 2-Propyl, 1-Butyl, 2-Butyl, 1-Pentyl, 2-Pentyl, 1-Hexyl, 2-Hexyl, 1-Heptyl, 2-Ethylhexyl und 1-Octyl, bevorzugt Methyl, Ethyl, 1-Propyl, 2-Propyl und 1-Butyl, besonders bevorzugt Methyl und Ethyl
oder Phenyl,
- R³ =: H, C₁-C₄-Alkyl wie Methyl, Ethyl, 1-Propyl, 2-Propyl und 1-Butyl,
bevorzugt H, Methyl und Ethyl bedeuten.

Als Beispiele für Ester der Formel 1 sind die folgenden bevorzugten Verbindungen genannt:

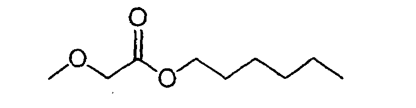

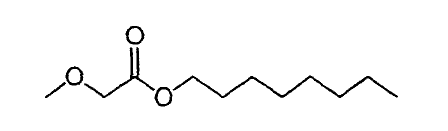

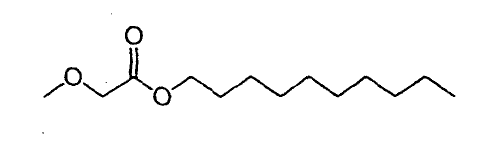

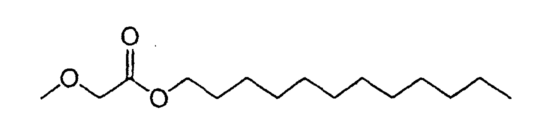

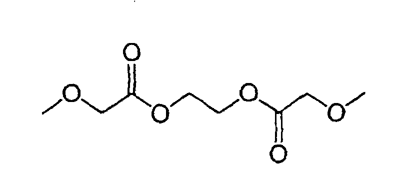

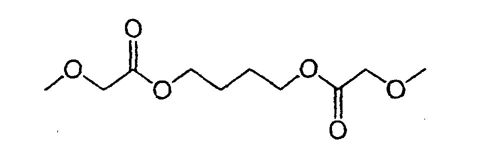

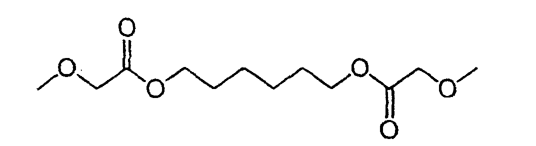

Das erfindungsgemäße Verfahren eignet sich zur Racematspaltung von alkoxysubstituierten primären Amin der allgemeinen Formel 2, 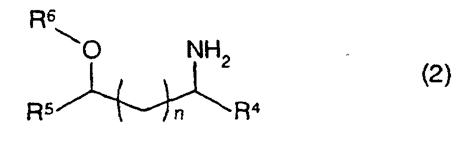 in der
- n =: 0 oder 1, bevorzugt 0,
- R⁴, R⁵: unabhängig voneinander H, C₁-C₈-Alkyl wie z.B. Methyl, Ethyl, 1-Propyl, 2-Propyl, 1-Butyl, 2-Butyl, 1-Pentyl, 2-Pentyl, 1-Hexyl, 2-Hexyl, 1-Heptyl, 2-Ethylhexyl und 1-Octyl
oder Phenyl,
bevorzugt H, C₁-C₄-Alkyl wie z.B. Methyl, Ethyl, 1-Propyl, 2-Propyl und 1-Butyl,
besonders bevorzugt H, Methyl und Ethyl,
- R⁶ =: C₁-C₆-Alkyl wie z.B. Methyl, Ethyl, 1-Propyl, 2-Propyl, 1-Butyl, 2-Butyl, 1-Pentyl, 2-Pentyl, 1-Hexyl und 2-Hexyl
oder Benzyl,
bevorzugt Methyl, Ethyl und Benzyl bedeuten.

Als Beispiele für Amine der Formel 2 ist die folgende Verbindungen als bevorzugt genannt:

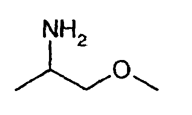

### Beispiel 1

Eine Mischung aus 1 mol-Äquivalent 2-Amino-1-methoxypropan und 1 mol-(bei Diestern 0,5 mol-) Äquivalente des jeweiligen Methoxyessigsäureesters wird mit 5 Gew.-% (bezogen auf das Amin) Novozym® 435 versetzt und 24 Stunden bei Raumtemperatur geschüttelt. Die Umsätze wurden gaschromatographisch bestimmt und sind in Tabelle 1 zusammengestellt:

**Tabelle 1:**

| Vergleich der Acylierungsgeschwindigkeit von 2-Amino-1-methoxypropan mit verschiedenen Methoxyessigsäureestern | | | | | |
|---|---|---|---|---|---|
| Zeit [h] | Methylester Umsatz [%] | Hexylester Umsatz [%] | Decylester Umsatz [%] | Ethandioldiester Umsatz [%] | 1,4-Butandioldiester Umsatz, [%] |
| 0,25 | 17,5 | 19,3 | 14,5 | 26,4 | 20,4 |
| 0,5 | 21,7 | 31,1 | 26,0 | 30,5 | |
| 0,75 | 24,0 | 37,4 | 33,1 | 41,6 | 34,7 |
| 1 | 25,9 | 41,0 | 37,8 | 45,0 | 38,9 |
| 1,5 | 28,1 | 46,1 | 43,8 | 49,2 | 44,1 |
| 2 | 29,4 | 48,8 | 47,7 | 51,4 | 47,0 |
| 2,5 | 30,2 | 50,7 | 49,7 | 52,9 | 48,9 |
| 3 | 31,1 | 51,6 | 51,1 | 54,0 | 50,2 |
| 5 | 33,3 | 53,4 | 53,6 | 57,7 | 53,4 |
| 6 | 33,9 | 54,0 | 54,3 | 58,2 | 54,0 |
| 24 | 39,2 | 63,2 | 63,9 | 66,3 | 62,1 |

### Beispiel 2

In einem auf 60°C beheizten Glasrohr (Innendurchmesser: 1 cm) wurden 2 g Novozym® 435 als Suspension im jeweiligen Ester vorgelegt. Anschließend wurde eine über Molekularsieb (4 Å) getrocknete äquimolare Mischung aus 2-Amino-1-methoxypropan und dem jeweiligen Ester mit konstanter Geschwindigkeit durch das Enzymbett gepumpt. Die dabei erzielten Umsätze sowie die Enantiomerenüberschüsse des langsamer reagierenden Enantiomers [(S)-1-Amino-2-methoxypropan] sind in Tabelle 2 zusammengestellt:

**Tabelle 2:**

| Vergleich der Umsätze und Enantiomerenüberschüsse bei der Racematspaltung von 1-Amino-2-methoxypropan mit verschiedenen Methoxyessigsäureestern | | | |
|---|---|---|---|
| Ester | Belastung [g/gh] | Umsatz [%] | ee [%] |
| Methoxyessigsäuremethylester | 7,5 | 3,1 | 3,0 |
| Methoxyessigsäureisopropylester | 7,5 | 54,9 | > 99,5 |
| Methoxyessigsäureisopropylester | 25,0 | 41,1 | 64,0 |
| Methoxyessigsäuredecylester | 25,0 | 61,5 | > 99,5 |

(Die Belastung entspricht der Menge der Amin/Ester-Mischung, die pro Gramm Novozym® 435 und Stunde durch das Enzymbett gepumpt wurde.)

## Patentansprüche

1. Verfahren zur Herstellung von optisch aktiven primären Aminen der Formel 2 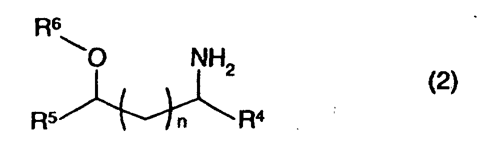 in der
n = 0 oder 1,
R⁴, R⁵ unabhängig voneinander H, C₁-C₈-Alkyl oder Phenyl,
R⁶ = C₁-C₆ Alkyl oder Benzyl bedeuten, durch
a) Umsetzung der primären Amine mit einem Ester der Formel 1 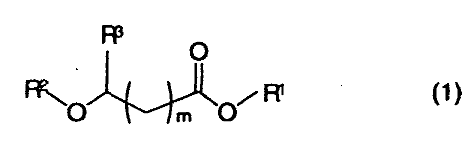 in der
m = 0 oder 1,
R¹ = verzweigtes oder unverzweigtes C₆-C₂₀-Alkyl oder Heteroalkyl mit 6 bis 20 Gerüstatomen, wobei der Alkyl oder Heteroalkylrest unabhängig voneinander durch 1 bis 5 Halogenatome und/oder eine Oxogruppe substituiert sein kann,
R² = C₁-C₈-Alkyl oder Phenyl,
R³ = H, C₁-C₄-Alkyl,
in Gegenwart einer Lipase und anschließender
b) Trennung des enantioselektiv acylierten Amins und des nicht umgesetzten Amins.

2. Verfahren zur Acylierung von primären Aminen der Formel 2 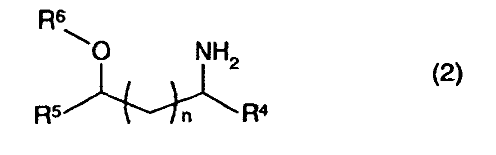 in der
n = 0 oder 1,
R⁴, R⁵ unabhängig voneinander H, C₁-C₈-Alkyl oder Phenyl,
R⁶ = C₁-C₆-Alkyl oder Benzyl,
durch Umsetzung der primären Amine mit einem Ester der Formel 1 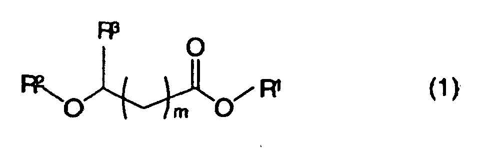 in der
m = 0 oder 1,
R¹ = verzweigtes oder unverzweigtes C₆-C₂₀-Alkyl oder Heteroalkyl mit 6 bis 20 Gerüstatomen, wobei der Alkyl oder Heteroalkylrest unabhängig voneinander durch 1 bis 5 Halogenatome und/oder eine Oxogruppe substituiert sein kann,
R² = C₁-C₈-Alkyl oder Phenyl,
R³ = H, C₁-C₄-Alkyl,
in Gegenwart einer Lipase.

3. Verfahren nach Anspruch 1, wobei man im Anschluß an Schritt b) oder c) das unerwünschte Enantiomer racemisiert oder unter Racemisierung hydrolysiert und in das Verfahren zurückführt.

4. Verfahren nach einem der Ansprüche 1 oder 3, wobei man die bei der Hydrolyse des acylierten Amins anfallende alkoxyoder phenoxysubstituierte Säure mit einem Alkohol R¹OH verestert, wobei R¹ die in Anspruch 1 angegebene Bedeutung hat, und in das Verfahren zurückführt.

5. Verfahren nach einem der Ansprüche 1 bis 4, wobei R¹ für ein verzweigtes oder unverzweigtes C₆-C₁₄-Alkyl oder Heteroalkyl mit 6 bis 14 Gerüstatomen, wobei der Alkyl oder Heteroalkylrest durch eine Oxogruppe substituiert sein kann.

6. Verfahren nach einem der Ansprüche 1 bis 5, wobei der Ester ausgewählt ist aus der Gruppe bestehend aus:

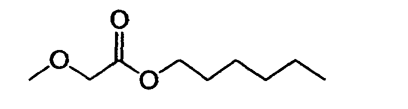

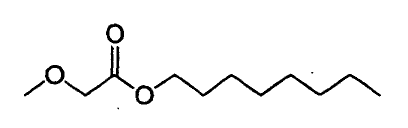

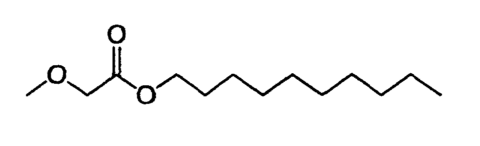

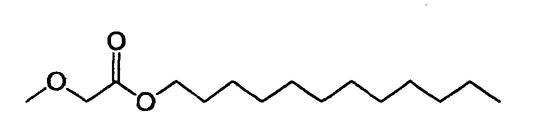

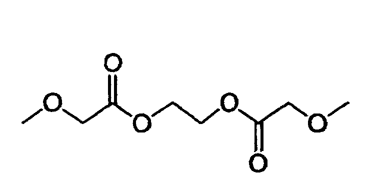

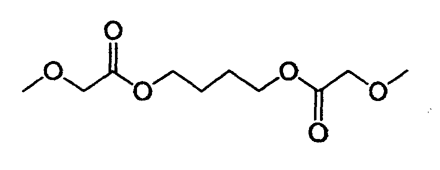

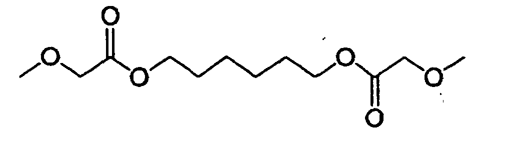

7. Verfahren nach einem der Ansprüche 1 bis 6, wobei das primäre Amin 2-Amino-1-methoxypropan ist.

8. Verfahren nach einem der Ansprüche 1 bis 6, wobei das acylierte Amin die (R)-Konfiguration aufweist und das nicht umgesetzte Amin die (S)-Konfiguration am Aminokohlenstoff aufweist.

9. Verfahren nach einem der Ansprüche 1 bis 8, wobei die Einsatzstoffe getrocknet und säurefrei vorliegen.

## Claims

1. A process for preparing optically active primary amines of the formula 2 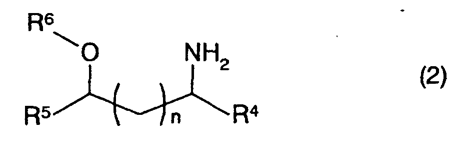 in which
n = 0 or 1,
R⁴, R⁵ = independently of one another H, C₁-C₈-alkyl or phenyl,
R⁶ = C₁-C₆-alkyl or benzyl,
by
a) reacting the primary amines with an ester of the formula 1 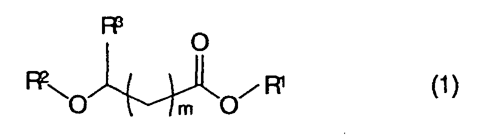 in which
m = 0 or 1,
R¹ = branched or unbranched C₆-C₂₀-alkyl or heteroalkyl having 6 to 20 backbone atoms, it being possible for the alkyl or heteroalkyl radical to be substituted, independently of one another, by 1 to 5 halogen atoms and/or an oxo group,
R² = C₁-C₈-alkyl or phenyl,
R³ = H, C₁-C₄-alkyl,
in the presence of a lipase and subsequently
b) separating the enantioselectively acylated amine and the unreacted amine.

2. A process for acylating primary amines of the formula 2 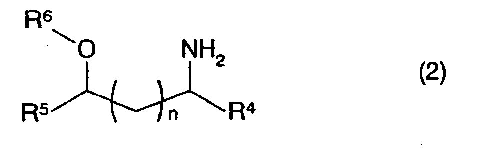 in which
n = 0 or 1,
R⁴, R⁵ = independently of one another H, C₁-C₈-alkyl or phenyl,
R⁶ = C₁-C₆-alkyl or benzyl,
by reacting the primary amines with an ester of the formula 1 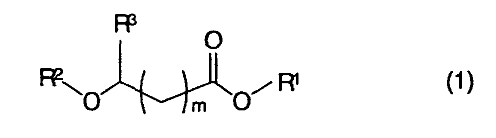 in which
m = 0 or 1,
R¹ = branched or unbranched C₆-C₂₀-alkyl or heteroalkyl having 6 to 20 backbone atoms, it being possible for the alkyl or heteroalkyl radical to be substituted, independently of one another, by 1 to 5 halogen atoms and/or an oxo group,
R² = C₁-C₈-alkyl or phenyl,
R³ = H, C₁-C₄-alkyl,
in the presence of a lipase.

3. A process as claimed in claim 1, wherein step b) or c) is followed by the unwanted enantiomer being racemized or hydrolyzed with racemization and returned to the process.

4. A process as claimed in either of claims 1 and 3, wherein the alkoxy- or phenoxy-substituted acid resulting from hydrolysis of the acylated amine is esterified with an alcohol R¹OH where R¹ has the meaning stated in claim 1, and returned to the process.

5. A process as claimed in any of claims 1 to 4, wherein R¹ is a branched or unbranched C₆-C₁₄-alkyl or heteroalkyl having 6 to 14 backbone atoms, it being possible for the alkyl or heteroalkyl radical to be substituted by an oxo group.

6. A process as claimed in any of claims 1 to 5, wherein the ester is selected from the group consisting of:

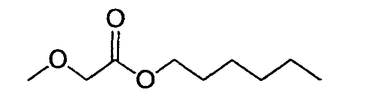

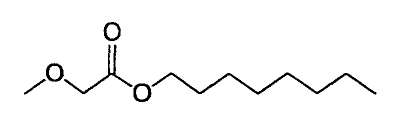

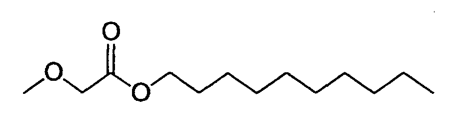

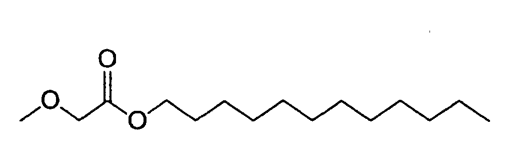

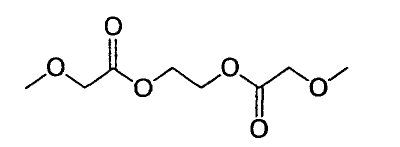

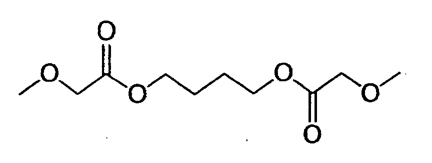

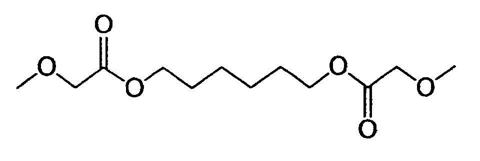

7. A process as claimed in any of claims 1 to 6, wherein the primary amine is 2-amino-1-methoxypropane.

8. A process as claimed in any of claims 1 to 6, wherein the acylated amine has the (R) configuration and the unreacted amine has the (S) configuration at the amino carbon.,

9. A process as claimed in any of claims 1 to 8, wherein the starting materials are in dried and acid-free form.

## Revendications

1. Procédé pour la préparation des isomères optiques d'amines primaires de formule (2) 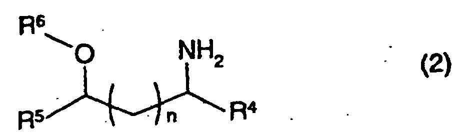 dans laquelle
n égal 0 ou 1,
R⁴, R⁵ représentent chacun, indépendamment l'un de l'autre, l'hydrogène, un groupe alkyle en C1-C8 ou phényle
R⁶ représente un groupe alkyle en C1-C6 ou benzyle, consistant
a) à faire réagir les amines primaires avec un ester de formule (1) 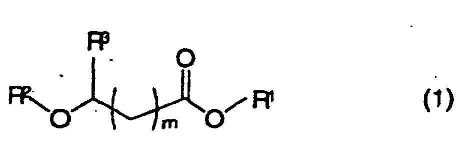 dans laquelle
m égal 0 ou 1,
R¹ représente un groupe alkyle à chaîne droite ou ramifiée en C6-C20 ou un groupe hétéroalkyle contenant 6 à 20 atomes de squelette, ces groupes alkyle et hétéroalkyle pouvant être substitués chacun, indépendamment l'un de l'autre, par 1 à 5 atomes d'halogènes et/ou un groupe oxo,
R² représente un groupe alkyle en C1-C8 ou phényle,
R³ représente l'hydrogène ou un groupe alkyle en C1-C4,
En présence d'une lipase, puis
b) à séparer l'amine qui a subi l'acylation énantiosélective et l'amine non convertie.

2. Procédé pour l'acylation d'amines primaires de formule (2) 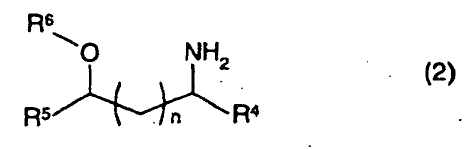 dans laquelle
n égal 0 ou 1,
R⁴, R⁵ représentent chacun, indépendamment l'un de l'autre, l'hydrogène, un groupe alkyle en C1-C8 ou phényle,
R⁶ représente un groupe alkyle en C1-C6 ou benzyle,
par réaction des amines primaires avec un ester de formule (1) 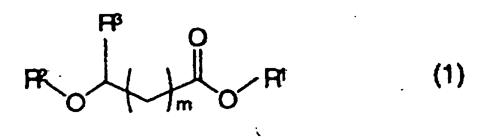 dans laquelle
m égal 0 ou 1,
R¹ représente un groupe alkyle à chaîne droite ou ramifiée en C6-C20 ou un groupe hétéroalkyle contenant 6 à 20 atomes de squelette, ces groupes alkyle et hétéroalkyle pouvant être substitués chacun, indépendamment l'un de l'autre, par 1 à 5 atomes d'halogène et/ou un groupe oxo,
R² représente un groupe alkyle en C1-C8 ou phényle,
R³ représente H ou un groupe alkyle en C1-C4,
en présence d'une lipase.

3. Procédé selon la revendication 1, dans lequel, après exécution du stade b) ou du stade c), on racémise l'énantiomère indésirable ou bien on l'hydrolyse avec racémisation et on le recycle dans les opérations.

4. Procédé selon l'une des revendications 1 ou 3, selon lequel on estérifie par un alccol R¹OH, R¹ ayant les significations indiquées dans la revendication 1, l'acide à substituant alcoxy ou phénoxy obtenu à l'hydrolyse de l'amine acylée, et on le recycle dans les opérations.

5. Procédé selon l'une des revendications 1 à 4, selon lequel R¹ représente un groupe alkyle à chaîne droite ou ramifiée en C6-C14 ou hétéroalkyle contenant 6 à 14 atomes de squelette, ces groupes alkyle et hétéroalkyle pouvant être substitués par un groupe oxo.

6. Procédé selon l'une des revendications 1 à 5, selon lequel l'ester est choisi dans le groupe consistant en les suivants :

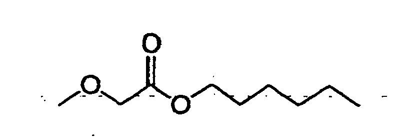

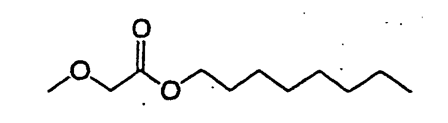

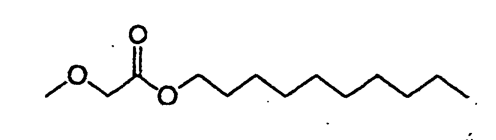

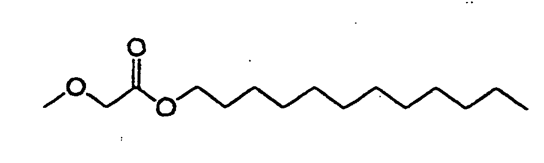

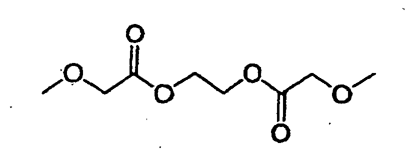

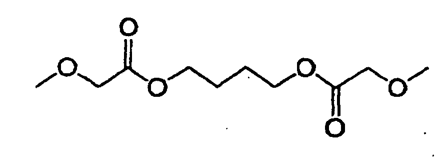

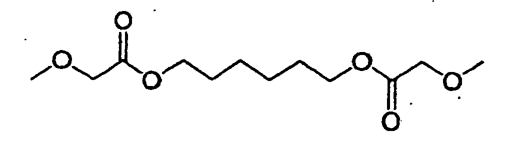

7. Procédé selon l'une des revendications 1 à 6, selon lequel l'amine primaire est le 2-amino-1-méthoxypropane.

8. Procédé selon l'une des revendications 1 à 6, selon lequel l'amine acylée est en configuration (R) et l'amine non convertie est en configuration (S) sur le carbone du groupe amino.

9. Procédé selon l'une des revendications 1 à 8, selon lequel les composants de départ sont à l'état sec et exempts d'acidité.
